(19) **Europäisches Patentamt**
**European Patent Office**
**Office européen des brevets**

(11) Veröffentlichungsnummer: **0 055 835**
**B1**

(12) **EUROPÄISCHE PATENTSCHRIFT**

(45) Veröffentlichungstag der Patentschrift:
**10.04.85**

(51) Int. Cl.⁴: **C 12 N 5/00** // C12N7/00,
A01N63/00

(21) Anmeldenummer: **81110440.5**

(22) Anmeldetag: **15.12.81**

(54) **Verfahren zur Züchtung von Insektenzellen.**

(30) Priorität: **20.12.80 DE 3048289**

(43) Veröffentlichungstag der Anmeldung:
**14.07.82 Patentblatt 82/28**

(45) Bekanntmachung des Hinweises auf die Patenterteilung:
**10.04.85 Patentblatt 85/15**

(84) Benannte Vertragsstaaten:
**AT BE CH DE FR GB IT LI NL SE**

(56) Entgegenhaltungen:
**DE - A - 2 355 354**

**CHEMICAL ABSTRACTS, Band 93, Nr. 25, 22. Dezember 1980, Zusammenfassung 235656c COLUMBUS, OHIO (US)**
**A. Mayr et al.: "Virologische Arbeitsmethoden" VEB Gustav Fisher Verlag, 1974 JENA (DD) 3.3. "Kultivierung von Arthropodenzellen" 3.3.1. "Insektenzellkulturen" Seiten 140-144**

(73) Patentinhaber: **HOECHST AKTIENGESELLSCHAFT, Postfach 80 03 20, D-6230 Frankfurt am Main 80 (DE)**

(72) Erfinder: **Röder, Anton, Dr., In den Bleichwiesen 9, D-6233 Kelkheim (Taunus) (DE)**

## Beschreibung

Im Zusammenhang mit der öffentlichen Diskussion über Umweltbeeinflussung durch chemische Schädlingsbekämpfung haben die Methoden der biologischen Schädlingsbekämpfung an Bedeutung zugenommen. Eine der erfolgversprechendsten Methoden ist die Verwendung von hochspezifischen insektenpathogenen Viren, insbesondere von Kernpolyederviren (NPV) und Granuloseviren (GV) aus der Gruppe der Baculoviren. Diese werden ähnlich wie chemische Pestizide in die Umgebung ausgebracht und von spezifischen Wirtstieren aufgenommen. Nur in diesen Wirtstieren verursachen sie eine Virusinfektion, die zum baldigen Tod führt. Eine Reihe von NPV- und GV-Präparaten befindet sich bereits im Handel bzw. in der praktischen Erprobung (vgl. Shieh u. Bohmfalk, Production and Efficiency of Baculoviruses, Biotechnology and Bioengineering Vol. XXII, 1357 ff. (1980).

Die Gewinnung ausreichender Mengen an Virusmaterial stösst jedoch auf praktische Probleme, die bisher noch nicht befriedigend gelöst sind. Grundsätzlich stehen zwei Wege zur Verfügung (a.a.O., 1361 ff.):

1. Vermehrung einer grossen Mengen von Wirtstieren, künstliche Infektion mit Virus und Isolation des Virus aus den gestorbenen Tieren;

2. Züchtung von Einzelzellen der Insekten in vitro in geeigneten Nährmedien, Infektion dieser Zellen und Aufarbeitung des Virusmaterials.

Das unter 1. beschriebene Verfahren liefert zwar grosse Mengen Virusmaterial, hat jedoch den Nachteil, dass die Wirtstiere nur zu bestimmten Jahreszeiten verfügbar sind und das Virusmaterial mit Insektenrückständen und mikrobiellen Verunreinigungen kontaminiert ist. Eine Reinigung ist prinzipiell zwar möglich, würde aber die Produktion stark verteuern.

Die unter 2. beschriebene Methode vermeidet diese Schwierigkeiten; sie steht und fällt jedoch mit der Verfügbarkeit eines geeigneten Kulturmediums. Die hierfür vorgeschlagenen und verwendeten Zusammensetzungen enthalten in der Regel anorganische Salze, Vitamine, Aminosäuren, Zucker und eine Reihe anderer organischer Verbindungen, die für die Zellphysiologie von Bedeutung sind; allen gemeinsam ist ausserdem ein Zusatz von ca. 5 bis 20% fötalem Kälberserum (FKS), das für das Zellwachstum unerlässlich ist. Typische Zusammensetzungen sind z.B. in J. Invertebrate Pathology 25, 363–370 (1975) beschrieben.

Als limitierender Faktor erweist sich dabei das FKS, das sehr teuer ist und nur in begrenzten Mengen zur Verfügung steht. Eine Produktion von Insektenzellen in technischem Massstab, die ihrerseits Voraussetzung ist für die technische Produktion von insektenpathogenen Viren, ist unter diesen Voraussetzungen nicht möglich. Es bestand daher ein erhebliches Bedürfnis, FKS durch eine billigere und ausreichend verfügbare Komponente zu ersetzen.

Überraschend wurde nun gefunden, dass man an Stelle des FKS vorteilhaft Eidotter verwenden kann.

Gegenstand der Erfindung ist somit ein Verfahren zur Züchtung und Vermehrung von Zellen, insbesondere Insektenzellen, unter Verwendung bekannter anorganischer Salze, Aminosäuren, Zucker, Vitaminen und andere organische Substanzen enthaltender Nährmedien, das dadurch gekennzeichnet ist, dass diese einen Zusatz von Eidotter enthalten.

Der Eidottergehalt beträgt 0,1 bis 5, vorzugsweise 0,5 bis 2 Gewichtsprozent der Nährlösung. Er kann FKS ganz oder auch teilweise ersetzen.

Ferner ist Gegenstand der Erfindung ein Nährmedium zur Züchtung und Vermehrung der Zellen, insbesondere Insektenzellen, gekennzeichnet durch einen Gehalt an Eidotter sowie die Verwendung von Eidotter als Zusatz zum Nährmedium bei der Züchtung und Vermehrung von Zellen, insbesondere Insektenzellen.

Zur Herstellung eines für die Zellzüchtung geeigneten Nährmediums benötigt man nur einen Bruchteil der erforderlichen FKS-Menge an Eidotter, nämlich ca. 5–20%. Es ist nicht erforderlich, den Eidotter vom Eiweiss zu trennen; geeignet sind vielmehr auch z.B. geschlagene oder gequirlte Eier. Vorzugsweise verwendet man jedoch käufliche Eidotteremulsion.

Auch die Herkunft des Eidotters ist von untergeordneter Bedeutung. Aus Gründen der leichten Verfügbarkeit eignet sich besonders Eidotter vom Hühnerei, doch kommen auch andere Eier, z.B. Enten- oder Gänseeier, als Eidotterquelle in Betracht.

Das erfindungsgemässe Verfahren eignet sich zur Züchtung einer Vielzahl von Insekten-Zellinien, z.B. solchen aus den Ordnungen Diptera und Lepidoptera. Genannt seien z.B. Zellinien aus Spodoptera frugiperda, Lymantria dispar, Mamestra brassicae, Trichoplusia ni, Ädes albopictus und Ädes ägypti. Diese wiederum eignen sich zur Vermehrung zahlreicher insektenpathogener Viren, z.B. Parvoviren, Poxviren, Baculoviren und Rhabdoviren, von denen besonders Kernpolyederviren wie diejenigen aus Autographa spp., Spodoptera spp. und Lymantria spp. von Interesse sind.

Das Verfahren ist nachstehend am Beispiel der Züchtung eines etablierten Zellstamms von Spodoptera frugiperda (Sf) beschrieben:

Zur Züchtung von den Insektenzellen wurde das käufliche Kulturmedium TC 10 (bestehend aus einer Salzlösung, Glukose, definierten Mengen an Aminosäuren, Vitaminen und einem Proteinhydrolysat [Tryptoseboullion]) in einem Versuch mit 10% FKS versetzt; in einem zweiten Versuch wurde die FKS-Konzentration auf 5% reduziert und eine 0,5%ige Eidotteremulsion (Fa. Oxoid) zugegeben. Die Zellzüchtung erfolgte in Monolayerflaschen (3 ml), Schüttelkulturen (20 ml) und Rührflaschen (500 ml).

Der Zusatz erhöhte die Zellausbeute auf das Doppelte (von $1,4 \times 10^6$ auf $2,8 \times 10^6$ Zellen/ml, verglichen mit einem Kontrollansatz mit 10% FKS.

In weiteren Versuchen wurde das Serum ganz weggelassen und unterschiedliche Eidotterkonzentrationen verwendet. Bei einem Zusatz von 1% Eidotter konnte FKS voll ersetzt werden, die Zellausbeuten bewegten sich in der gleichen Grössenordnung wie bei Verwendung von 10% FKS (Tabelle 1).

Tabelle 1
Zellausbeute bei Zusatz von FKS bzw. von Eidotteremulsion im Monolayer

| | Zellausbeute (Zellen/ml) bei Verwendung von TC/10 | |
| | + 10% FKS | + 1% Eidotter-emulsion |
| --- | --- | --- |
| Versuch 1 | $1,4 \times 10^6$ | $1,4 \times 10^6$ |
| Versuch 2 | $2,0 \times 10^6$ | $1,7 \times 10^6$ |
| Versuch 3 | $1,4 \times 10^6$ | $1,8 \times 10^6$ |

Vergleichbare Ergebnisse wurden bei Verwendung von Suspensionszellen in Schüttel- und Rührsystemen erhalten. In Langzeitversuchen wurde festgestellt, dass das Zellwachstum in dem erfindungsgemässen Kulturmedium über 60 Passagen hinaus stabil bleibt.

Die mit Eidotterzusatz in FKS-freiem Medium gezüchteten Zellen wurden regelmässig als Substrat für die Produktion insektenpathogener Kernpolyederviren verwendet. Die Virusausbeute blieb im Vergleich zu Kontrollansätzen mit FKS-Zusatz unverändert (Tabelle 2):

Tabelle 2
Polyederausbeute im Monolayersystem

| | TC 100 + | |
| | + 10% FKS | + 1% Eidotter |
| --- | --- | --- |
| PIB/ml (polyhedral inclusion bodies) | $1,3 \times 10^7$ | $1,2 \times 10^7$ |
| | $1,9 \times 10^7$ | $1,6 \times 10^7$ $1,3 \times 10^7$ |
| | (2 Versuche) | (3 Versuche) |

Elektronenoptische Untersuchungen sowie Infektionsversuche in vitro zeigten, dass die Morphologie sowie die biologische Aktivität der produzierten Viren unverändert war.

**Patentansprüche**

1. Verfahren zur Züchtung und Vermehrung von Zellen, insbesondere Insektenzellen, durch Kultivierung derselben in an sich bekannten Nährmedien, dadurch gekennzeichnet, dass man dem Nährmedium Eidotter zusetzt.

2. Verfahren nach Anspruch 1, dadurch gekennzeichnet, dass der Zusatz an Eidotter 0,1 bis 5 Gewichtsprozent des Nährmediums beträgt.

3. Verfahren nach Anspruch 1, dadurch gekennzeichnet, dass der Zusatz an Eidotter 0,5 bis 2 Gewichtsprozent des Nährmediums beträgt.

4. Nährmedium zur Züchtung und Vermehrung von Zellen, insbesondere Insektenzellen, gekennzeichnet durch einen Gehalt an Eidotter.

5. Nährmedium gemäss Anspruch 4, dadurch gekennzeichnet, dass der Eidottergehalt 0,1 bis 5 Gewichtsprozent beträgt.

6. Nährmedium gemäss Anspruch 4, dadurch gekennzeichnet, dass der Eidottergehalt 0,5 bis 2 Gewichtsprozent beträgt.

7. Verwendung von Eidotter als Zusatz zum Nährmedium bei der Züchtung und Vermehrung von Zellen, insbesondere Insektenzellen.

**Revendications**

1. Procédé de multiplication de cellules, en particulier de cellules d'insectes, par culture dans des milieux de culture connus, procédé caractérisé en ce que l'on ajoute du jaune d'œuf au milieu de culture.

2. Procédé selon la revendication 1, caractérisé en ce que l'addition de jaune d'œuf est de 0,1 à 5% du poids du milieu de culture.

3. Procédé selon la revendication 1, caractérisé en ce que l'addition de jaune d'œuf est de 0,5 à 2% du poids du milieu.

4. Milieu de culture pour la multiplication de cellules, en particulier de cellules d'insectes, caractérisé en ce qu'il contient du jaune d'œuf.

5. Milieu de culture selon la revendication 4, caractérisé en ce qu'il contient 0,1 à 5% en poids de jaune d'œuf.

6. Milieu selon la revendication 4 caractérisé en ce qu'il contient 0,5 à 2% en poids de jaune d'œuf.

7. L'emploi de jaune d'œuf comme additif à un milieu de culture pour la mumtiplication de cellules, en particulier de cullules d'insectes.

**Claims**

1. A method for the cultivation and reproduction of cells, especially insect cells by cultivation thereof in known nutrient media, in which egg yolk is added to the nutrient medium.

2. The method as claimed in Claim 1, in which from 0,1 to 5 weight %, relative to the nutrient medium, of egg yolk is added.

3. The method as claimed in Claim 1, in which from 0,5 to 2 weight % of egg yolk, relative to the nutrient medium is added.

4. Nutrient medium for the cultivation and reproduction of cells, especially insect cells, which comprises a content of egg yolk.

5. The nutrient medium as claimed in Claim 4, wherein the content of egg yolk is from 0,1 to 5 weight %.

6. The nutrient medium as claimed in Claim 4, wherein the content of egg yolk is from 0,5 to 2 weight %.

7. Use of egg yolk as additive for nutrient media for the cultivation and reproduction of cells, especially insect cells.